Europäisches Patentamt

European Patent Office

Office européen des brevets

(19)

(11) Publication number: **0 200 061**
**A2**

(12) # EUROPEAN PATENT APPLICATION

(21) Application number: **86105026.8**

(22) Date of filing: **11.04.86**

(51) Int. Cl.⁴: **A 61 B 5/08**
**A 61 B 5/10**

(30) Priority: **29.04.85 SE 8502070**

(43) Date of publication of application:
**05.11.86 Bulletin 86/45**

(84) Designated Contracting States:
**AT BE CH DE FR GB IT LI LU NL**

(71) Applicant: **Mediinvent HB**
**Protokollgränden 38**
**S-222 47 Lund(SE)**

(72) Inventor: **Claren, Jan**
**Protokollgränden 38**
**S-222 47 Lund(SE)**

(72) Inventor: **Felding, Anders**
**Ö Förstadsgatan 21**
**S-211 31 Malmö(SE)**

(54) **Apparatus for monitoring respiration of a subject.**

(57) An apparatus for continuous monitoring of the respiration of a subject (2), which comprises an elastic or flexible leader-element (1) being adapted to be located around the body of the subject to receive respiration-movements and to transform these to electric signals to be received by a sensing-device connected to said leader-element.

The sensing-device is connected to an alarm-device (7,8) which preferably is activated by a micro-processor not until a predetermined time has elapsed since a ceasing or strong weakening of the electric signals.

EP 0 200 061 A2

Croydon Printing Company Ltd.

## APPARATUS FOR MONITORING RESPIRATION OF A SUBJECT

This invention relates to an apparatus for monitoring the respiration of a subject such as a person. Said apparatus comprises an electric circuit including means for providing electric signals in reponse to movements of respiration, i.e. body-movements caused by the respiration, and a sensing-unit for these signals. The invention is particularly concerned with an apparatus of the above-mentioned kind for continous monitoring of the respiration of an infant.

Every year infants die in so-called sudden infant death (SID) due to the fact that the respiration often for unknown reasons ceases. The course is accute and leads, if untreated, to death already within a few seconds.

The need for an effective and reliable apparatus for monitoring the respiration of in particular an infant is therefor great.

An object of the invention is thus to provide such an apparatus, and this object is achieved in accordance with the invention by means of an apparatus as defined in the accompanying claims.

In accordance with the invention is thus provided an apparatus for continous monitoring of the respiration of a subject, in particular an infant, comprising an electric circuit including means for providing electric signals in response to respiration-
-movements of the subject, and a sensing-unit for said signals. This apparatus is characterized in that said means comprises an elastic or flexible leader-element which is adapted to receive said movements, whwreby said electric signals in the circuit are created through resistance-alterations in the so influenced element.

The expression "adapted to receive said movements" as regards the leader-element means that said element is adapted to resiliently expand an contract, respectively, under influence from the respiration-movements. This necessarily means that the elastic or

flexible leader-element should be in contact with the
monitored subject in such a way that the mechanical transferring of the said movements to the element is secured when
the apparatus is in use.

In accordance with a simple embodiment of the invention, said
elastic or flexible leader-element may be in form of a thin
thread or a similar elongated member, e.g. a narrow strip,
adapted to be located around the body of the subject to be
monitored, preferably around the chest thereof.

A cheap and effective elongated leader-element may be a thin
rubber thread, such as silicon, comprising embedded therein
an electrically leading material such as carbon particles.
The invention is however not restricted as regards the choice
of material for the said leader-element, other than that the
material which is chosen shall be elastic or flexible and
electrically leading.

Conveniently, said leader-element is provided with a clasping
device for releasable clasping and retaining of the element
in place around the monitored subject.

In accordance with a particularly preferred embodiment of the
invention the sensing-unit is connectable to an alarm-device
which is adapted to yield an alarm (visible and/or audible)
when the electric signals sensed by the sensing-device have
ceased or strongly weakened. Preferably, said sensing-device
is connectable to such an alarm-device via a micro-processor
which in advance may be programmed to activate the alarm when
a certain (optional) time has elapsed since ceasing or strong
weakening of the electric signals.

In connection with infants, which naturally show an alternating
respiration-rhythm (with from some up to ten seconds of spaces
between consecutive respirations) the microprocessor may be
programmed such that it will not activate the alarm-device until for example about 10 s have passed since the sensing-
device sensed the previous electric signal. Thereby, "false"
alarms every time the monitored infant makes a natural breathing-
space are avoided.

A compact and easily handled apparatus is provided in accordance
with the invention when the sensing-unit, micro-processor, and
alarm-device are hosed within one and same housing, for example
a plastic box of the size of a match-box, fitted with contact
members for electric connection to the separate leader-element.
Within the said hosing or plastic box may be incorporated a
battery, preferably a rechargable battery, whereby the hosing
or plastic box preferably comprises a corresponding contact
member for connection with a recharging device for the battery.

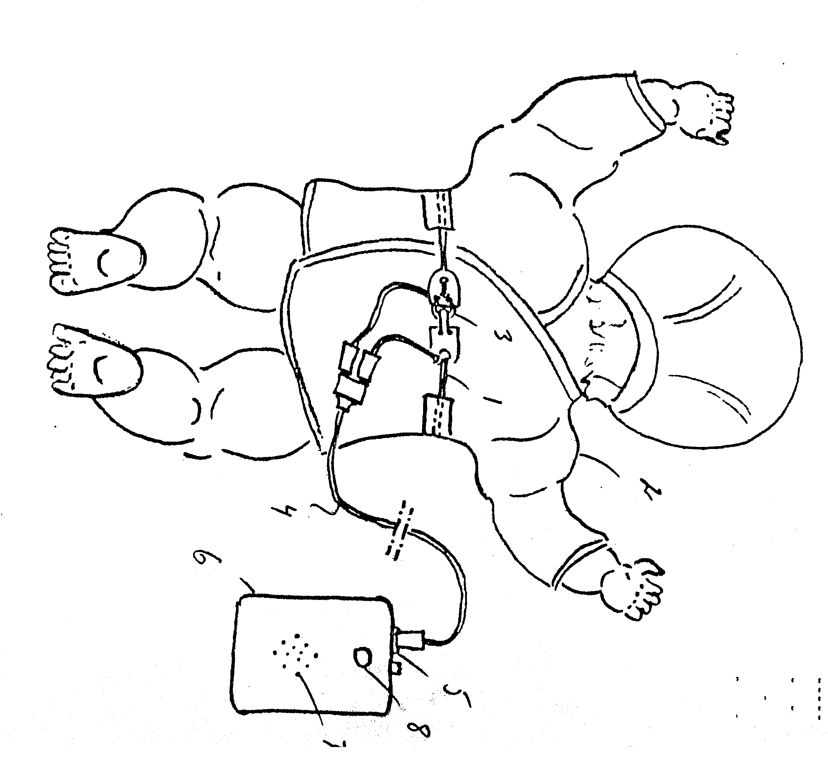

The present invention will be further described in the following
with reference to the accompanying drawing illustrating the use
of the present apparatus according to a preferred embodiment.

As shown in the drawing, the present apparatus preferably comprises an elastic or flexible leader-element in form of a thin
(about 1 mm) thread which may be manufactured from for example
silicon-rubber having electrically particles of carbon embedded
therein.

The thread 1 is adapted to be located around the chest of the
subject to be monitored, such as an infant 2, to receive (i.e.
to alternatingly expand an contract) respiratory movements and
to provide electric signals in response to the so received
movements, as explained hereinabove.

The thread 1 is further provided with a clasp 3 by means of
which the thread 1 releasably, yet safetely, is maintained in
place around the body, preferably around the chest, as shown.

Finally, the thread 1 is through a leader 4 connectable to a contacting-member 5 of a plastic box 6 for connection with a (not shown) sensing-unit located within said box 6 for receiving the electric signals from the thread 1.

The plastic box 6 further houses an alarm-device 7 and 8, a (not shown) micro-circuit, and a rechargable battery (not shown) which is adapted to ~~fædd~~ feed the thread 1 with an electric current.

The said micro-circuit in the plastic box 6 is in advance so set, that it will activate an alarm (loud-speaker 7 and lamp 8) of the alarm-device, when a predetermined time, for example 10 s, has elapsed since the previous electric signal from the thread was received, as explained above.

Claims

1. Apparatus for continous monitoring of respiration of a
subject (2), which comprises an electric circuit including
means (1) for providing electric signals in response to
respiration-movements of the subject, and a sensing-device
for receiving said signals, <u>characterized</u> in that said
means comprises an elastic or flexible leader-element which
is adapted to receive the respiration-movements, whereby
the electric signals in the circuit are created due to
resistance-alterations of the so influenced leader-element.

2. Apparatus in accordance with claim 1, <u>characterized</u> in
thatthe electricx leader-element (1) is in form of a thin
thread manufactured from a silicon-rubber and having electrically leading particles embedded therein.

3. Apparatus in accordance with claim 1 or 2, <u>characterized</u>
in that the electric leader-element (1) is provided with a
clasping device (3) for releasable clasping of the said
element (1) in position around the subject (2).

4. Apparatus in accordance with any of the preceding claims,
<u>characterized</u> in that the sensing-device is connectable to
an alarm-device (6,7) which is adapted to yield an alarm
in response to a ceasing or strong weakening of the said
electric signals.

5. Apparatus in accordance with claim 4, <u>characterized</u> in that
the sensing-device is connectable to an alarm-device via a
programable micro-processor.

6. ẍ. Apparatus in accordance with claim 5, <u>characterized</u> in
that the ensing-device, alarm-device and micro-processor are
housed within one and same housing (6) which is provided with

0200061

contacting means (5) for connection with the elastic or flexible leader-element (1).

7. Apparatus in accordance with claim 6, <u>characterized</u> in that the housing (6) houses a battery which is adapted to feed the electric circuit with electric current.